# FASCICULE DE BREVET EUROPEEN

(11) **EP 1 147 693 B1**
(45) Date de publication et mention de la délivrance du brevet: **26.07.2006**
(21) Numéro de dépôt: 99961999.2
(22) Date de dépôt: 20.12.1999
(51) Int. Cl.: H05H 7/00, G21K 5/10

(54) **DISPOSITIF DE TRAITEMENT D'UN VOLUME CIBLE PAR UN FAISCEAU DE PARTICULES**
VORRICHTUNG ZUR BEHANDLUNG EINES ZIELVOLUMENS DURCH EINEN TEILCHENSTRAHL
DEVICE FOR TREATING A TARGET VOLUME WITH A PARTICLE BEAM

(30) Priorité: 24.12.1998 BE 9800935
(43) Date de publication de la demande: 24.10.2001
(73) Titulaire: ION BEAM APPLICATIONS S.A., 1348 Louvain-la-Neuve (BE)
(72) Inventeur: JONGEN, Yves, B-1348 Louvain-La-Neuve (BE)
(74) Mandataire: Van Malderen, Joëlle
(86) Numéro de dépôt international: PCT/BE1999/000167
(87) Numéro de publication internationale: WO 2000/040064

(56) Documents cités:
- KANAI T ET AL: "Three-dimensional beam scanning for proton therapy" NUCLEAR INSTRUMENTS AND METHODS IN PHYSICS RESEARCH, 1 SEPT. 1983, NETHERLANDS, vol. 214, no. 2-3, pages 491-496, XP002114346 ISSN: 0167-5087 cité dans la demande
- DATABASE WPI Section EI, Week 198904 Derwent Publications Ltd., London, GB; Class X14, AN 1989-030447 XP002136197 & SU 1 362 388 A (SUKHOMLYNOV V F), 30 juillet 1988 (1988-07-30)
- PEDRONI E ET AL: "The 200-MeV proton therapy project at the Paul Scherrer Institute: conceptual design and practical realization" MEDICAL PHYSICS, JAN. 1995, USA, vol. 22, no. 1, pages 37-53, XP000505145 ISSN: 0094-2405 cité dans la demande

## Description

### Objet de l'invention

La présente invention se rapporte à un *dispositif* de traitement d'un volume cible par un faisceau de particules, notamment de protons.

Le domaine d'application est la protonthérapie utilisée en particulier dans le cas du traitement du cancer, où il est nécessaire de proposer un *dispositif* d'irradiation d'un volume cible constituant la tumeur à traiter.

### Etat de la technique

La radiothérapie est l'une des voies possibles pour le traitement du cancer. Elle se base sur l'irradiation du patient, plus particulièrement de sa tumeur, à l'aide de rayonnements ionisants. Dans le cas particulier de la protonthérapie, l'irradiation est réalisée à l'aide d'un faisceau de protons. C'est la dose de radiation ainsi délivrée à la tumeur qui est responsable de sa destruction.

Dans ce contexte, il importe que la dose prescrite soit effectivement délivrée au sein du volume cible défini par le radiothérapeute, tout en épargnant autant que possible les tissus sains et les organes critiques avoisinants. On parle de "conformation" de la dose délivrée au volume cible. En protonthérapie, on connaît différentes méthodes pouvant être utilisées à cet effet, qui sont regroupées en deux catégories : les méthodes dites passives et les méthodes dites actives.

Qu'elles soient actives ou passives, ces méthodes ont pour but commun de manipuler un faisceau de protons produit par un accélérateur de particules de manière à assurer la couverture complète du volume cible selon les trois dimensions : la "profondeur" (dans la direction du faisceau) et, pour chaque profondeur, les deux dimensions définissant le plan perpendiculaire au faisceau. Dans le premier cas, on parlera de "modulation" de la profondeur, ou encore de modulation du parcours des protons dans la matière, alors que dans le deuxième cas, on parlera de la mise en forme du champ d'irradiation dans le plan perpendiculaire au faisceau.

Les méthodes passives utilisent un dégradeur d'énergie pour ajuster le parcours des protons à leur valeur maximale, correspondant au point le plus profond de la zone à irradier, associé à une roue tournante d'épaisseur variable pour réaliser la modulation du parcours (ce dernier dispositif étant ainsi appelé modulateur de parcours). La combinaison de ces éléments avec un "compensateur de parcours" (ou encore "bolus") et un collimateur spécifique, permet d'obtenir une distribution de dose bien conforme à la partie distale du volume cible. Toutefois, un inconvénient majeur de cette méthode réside dans le fait que les tissus sains situés en aval de la partie proximale en dehors du volume cible sont eux aussi parfois soumis à des doses importantes. De plus, la nécessité d'utiliser un compensateur et un collimateur spécifique au patient et à l'angle d'irradiation alourdit la procédure et en augmente le coût.

Par ailleurs, en vue d'élargir les faisceaux étroits délivrés par l'accélérateur et le système de transport de faisceau, et ceci de manière à couvrir les grandes surfaces de traitement exigées par la radiothérapie, ces méthodes utilisent généralement un système composé d'un double diffuseur. Cependant, les protons perdent de l'énergie dans ces diffuseurs, et de grands- champs d'irradiation aux profondeurs les plus grandes sont dès lors difficiles à obtenir à moins de disposer d'une "réserve en énergie" par le biais de l'utilisation d'un accélérateur délivrant des protons d'une énergie bien supérieure à celle qui est nécessaire pour atteindre les zones les plus profondes à l'intérieur du corps humain. Or, il est bien connu que le coût de tels accélérateurs susceptibles de fournir des protons augmente proportionnellement avec l'énergie. Malgré ces inconvénients, les méthodes passives ont été largement utilisées dans le passé et le sont encore aujourd'hui. On peut citer comme exemple de méthode passive, la méthode dite de "double diffusion" bien connue dans l'état de la technique *(Chu W.T. et al. , Rev. Sci. Instr. , Vol. 64, No. 8, ISSN 0034-6748, p. 2055-2122 (Aug. 1993)*; voir pp 2080-2083).

Les méthodes dites actives ont pour but de résoudre certains ou parfois même tous les problèmes liés aux méthodes passives. Il existe en fait plusieurs types de méthodes actives. Une première série d'entre elles utilisent une paire d'aimants pour balayer le faisceau sur une surface circulaire ou rectangulaire. C'est le cas par exemple des méthodes dites de "wobbling" et de "raster scanning". Selon certaines de ces méthodes, le faisceau balayé est modulé par un modulateur de parcours similaire à ceux utilisés dans les méthodes passives. On continue à utiliser, dans ce cas, des collimateurs fixes et des compensateurs de parcours. Selon d'autres méthodes, le volume à traiter est découpé en plusieurs tranches successives, correspondant à des profondeurs successives. Chaque tranche est ensuite balayée par le faisceau, à l'aide des deux aimants de balayage, de manière à couvrir une surface dont les contours sont adaptés à la forme de la tumeur à traiter. Cette forme peut être différente pour chacune des tranches à traiter et est définie à l'aide d'un collimateur variable composé de multiples lames mobiles. On connaît par W. Chu, B. Ludewigt et T. Renner (*Rev. Sci. Instr.* 64, pp. 2055 (1993)) un exemple de ce type de méthode. Grâce à ces méthodes, on peut traiter de grands champs d'irradiation, même aux points les plus profonds du volume à traiter. Cependant, il est parfois nécessaire, selon certaines formes de réalisation basées sur ces méthodes, de continuer à utiliser un bolus et un compensateur. Dans le cas des méthodes qui mettent en oeuvre le découpage par tranches, une meilleure conformation est obtenue entre la dose délivrée et le volume à traiter, pour chaque tranche. Cependant, il est nécessaire, pour chaque tranche d'irradiation, d'adapter le collimateur multi-lames au contour de la section du volume à traiter. La qualité de la conformation dépendra, bien entendu, de la "finesse" du découpage en tranches.

Pour s'affranchir de la nécessité d'utiliser des compensateurs et des collimateurs, même multi-lames, et pour obtenir la meilleure conformation possible de la dose délivrée au volume à traiter, une deuxième série de méthodes actives se sert des aimants de balayage pour définir le contour de la zone à irradier, pour chaque plan d'irradiation, et réalise un découpage à trois dimensions du volume à traiter en de multiples points. Comme pour la première famille de méthodes actives, le déplacement du faisceau selon la dimension longitudinale, dans la direction du faisceau, se fera soit en modifiant l'énergie au niveau de l'accélérateur, soit en utilisant un dégradeur d'énergie. Celui-ci pourra être situé à la sortie de l'accélérateur ou, à l'opposé, dans la tête d'irradiation, près du patient. Après découpage du volume à irradier en de nombreux petits volumes ("voxels"), chacun de ces volumes se voit délivrer la dose voulue à l'aide d'un fin faisceau balayé en trois dimensions. Les collimateurs spécifiques et autres compensateurs ne sont plus nécessaires. On connaît par E. Pedroni et al. (*Med. Phys.* 22(1) (1995)) un exemple de mise en oeuvre de ce principe. Selon cette réalisation, la dose est déposée par le balayage, selon les trois dimensions, d'un "sport" produit par un faisceau étroit. C'est la technique dite de "pencil beam scanning". La superposition d'un nombre très élevé de ces éléments de dose individuels, délivrés de façon statique, permet d'obtenir une conformation parfaite de la dose au volume cible. Selon cette réalisation, le changement de la position du spot se fait toujours avec le faisceau arrêté. Le déplacement le plus rapide du spot se fait à l'aide d'un aimant déflecteur (le "sweeper magnet"). Le mouvement selon le deuxième axe de balayage se fait à l'aide d'un dégradeur ("range shifter"), situé dans la tête d'irradiation, qui permet de balayer le spot selon la profondeur. Enfin, la troisième direction est parcourue grâce au mouvement de la table qui supporte le patient. La position et la dose correspondant à chaque spot sont prédéterminées à l'aide d'un système informatique de planification du traitement. Lors de chaque mouvement du faisceau; c'est-à-dire lors de chaque déplacement du spot, le faisceau est interrompu. Cela se fait à l'aide d'un aimant qui a pour mission de dévier le faisceau vers une direction autre que celle du traitement ("fast kicker magnet").

Ce mode de mise en oeuvre des méthodes dites actives apporte une solution aux problèmes rencontrés par les autres techniques citées précédemment, et il permet d'obtenir la meilleure conformation possible de la dose délivrée au volume à traiter. Cependant, il souffre également de quelques inconvénients. Premièrement, la nécessité d'interrompre le faisceau avant chaque changement de la position du spot a pour conséquence d'allonger considérablement la durée du traitement. Ensuite, le déplacement de la table sur laquelle se trouve le patient est généralement mal perçu par les radiothérapeutes, qui préfèrent éviter toute action pouvant avoir comme conséquence le mouvement des organes à l'intérieur du corps du patient. Enfin, l'utilisation du dégradeur ("range shifter") en aval, juste avant le patient, a pour effet de détériorer certaines des caractéristiques du faisceau.

On connaît également par G. Kraft et al. (*Hadrontherapy in Oncology,* U. Amaldi and B. Larsson, editors, Elsevier Science (1994)) un autre exemple de mise en oeuvre d'une méthode active, développée particulièrement pour les faisceaux d'ions lourds. Ici aussi, le volume à traiter est découpé en une série de tranches successives. Selon cette réalisation, le balayage en profondeur du spot, pour passer d'une tranche à l'autre, est effectué en changeant l'énergie du faisceau directement au niveau de l'accélérateur, qui est dans ce cas un synchrotron. Chaque tranche du volume à traiter est parcourue une seule fois par le spot, le balayage de celui-ci étant réalisé à l'aide de deux aimants de balayage, dans les directions X et Y (la direction Z étant celle du faisceau, dans le sens de la profondeur). Le balayage se fait sans interruption du faisceau, à intensité constante. La vitesse de balayage est variable et est fixée en fonction de la dose à délivrer dans chaque élément de volume. Elle est également ajustée de manière à tenir compte des éventuelles fluctuations de l'intensité du faisceau. Ainsi, cette méthode permet de s'affranchir de la plupart des inconvénients liés aux méthodes décrites plus haut. Cependant, cette méthode a été spécialement développée pour des ions lourds produits par un synchrotron dont l'énergie peut être variée "pulse par pulse". De plus, ce système irradie une seule fois chaque tranche du volume à traiter, ce qui peut poser des problèmes en cas de mouvement d'organes en cours d'irradiation (par exemple lorsque le volume cible est affecté par la respiration).

Le document "Three-dimensional Beam Scanning for Proton Therapy" de Kanai et al. publié dans Nuclear Instruments and Methods in Physic Research (1^{er} septembre 1983), The Netherlands, Vol. 214, No. 23, pp. 491-496 décrit l'utilisation d'un synchrotron produisant un faisceau de protons contrôlé par des aimants de balayage, qui est ensuite dirigé vers un dégradeur d'énergie qui a pour but de modifier les caractéristiques en énergie du faisceau de protons. Ce dégradeur est essentiellement constitué par un bloc de matière dont l'épaisseur est variable de manière discrète. La dose de protons pour chaque volume cible est ajustée de manière dynamique par une mesure et un calcul en temps réel effectués à l'aide d'un ordinateur. Ceci permet d'obtenir une conformation de la dose à fournir en fonction du volume de la cible. On observe qu'aucune régulation du courant du faisceau n'est réalisée dans le procédé.

### Buts de l'invention

La présente invention vise à proposer un *dispositif* de traitement d'un volume cible par un faisceau de particules qui évite les inconvénients des méthodes décrites précédemment tout en permettant de délivrer une dose sur le volume cible avec le plus de flexibilité possible.

En particulier, la présente invention vise à proposer *un dispositif* de traitement qui permet d'obtenir un rapport variant de 1 à 500 pour la dose fournie pour chaque élément d'un volume cible.

La présente invention vise en particulier à proposer *un dispositif* qui s'affranchisse d'un grand nombre d'éléments auxiliaires tels que collimateurs, compensateurs, diffuseurs ou même modulateurs de parcours.

La présente invention vise en outre à proposer *un dispositif* qui permet de s'affranchir du mouvement du patient.

La présente invention vise également à proposer *un dispositif* qui permet d'obtenir une protection contre une absence d'émission du faisceau (blanc ou trou) ou contre l'arrêt du déplacement dudit faisceau.

### Eléments caractéristiques de la présente invention

La présente invention se rapporte à un dispositif de traitement d'un faisceau de particules produit par un accélérateur et visant à permettre l'irradiation d'un volume cible par un faisceau de particules, ledit dispositif comprenant un accélérateur de particules tel qu'un cyclotron permettant de générer un spot localisé dans le volume cible associé à des moyens de balayage permettant d'obtenir un balayage dudit spot dans les deux directions perpendiculaires à la direction du faisceau ainsi qu'à des moyens permettant d'obtenir une variation de l'intensité dudit faisceau de particules, caractérisé en ce qu' il comprend des moyens de commande adaptés à commander de manière simultanée les moyens de balayage et les moyens de variation de l'intensité du faisceau par un algorithme de planification et de régulation de manière à obtenir une conformation de la dose délivrée pour le volume cible.

De préférence, les moyens de balayage permettent le déplacement du spot dans les deux directions perpendiculaires à la direction du faisceau de manière à définir un plan d'irradiation.

Les moyens de balayage sont tels qu'ils permettent en outre un balayage de chaque plan d'irradiation plusieurs fois par le spot.

Avantageusement, le dispositif de l'invention comprend des moyens de variation de l'énergie du faisceau de particules permettant le déplacement du spot au sein du volume en passant d'un plan d'irradiation à l'autre selon la profondeur.

De préférence, les moyens de variation de l'énergie du faisceau de particules sont disposés immédiatement après l'extraction de l'accélérateur du faisceau de particules. Ces moyens de variation de l'énergie du faisceau de particules sont de préférence constitués par un dégradeur d'énergie.

Avantageusement, les moyens de balayage sont constitués par deux aimants de scanning si tués de préférence dans la tête d'irradiation.

En d'autres termes, les mouvements dans un plan d'irradiation sont effectués à l'aide de deux aimants situés de préférence dans la tête d'irradiation. Le mouvement du spot d'un plan d'irradiation à l'autre s'effectue en modifiant l'énergie du faisceau de particules à l'aide d'un dégradeur d'énergie.

La commande des aimants de balayage et des moyens de variation de l'intensité du faisceau est de préférence exécutée à l'aide d'un algorithme de planification et de régulation des trajectoires desdites particules en y associant une boucle de régulation de haut niveau corrigeant en temps réel lesdites trajectoires optimales pour obtenir une meilleure conformation de la dose au volume cible.

Avantageusement, un système informatique pour la mise en oeuvre de l'algorithme de planification et de traitement permet de déterminer la dose correspondant à chaque spot en prédéterminant l'intensité du faisceau et la vitesse de balayage pour chaque volume d'irradiation ou voxel.

De préférence, les moyens de balayage agissent en déplaçant le spot sans interruption du faisceau. En d'autres termes, le déplacement dans les deux directions perpendiculaires à la direction du faisceau se fait de manière continue.

On notera qu'en outre, les contours des surfaces dans chaque plan d'irradiation sont contrôlés par des éléments de balayage.

Enfin, le dispositif de l'invention peut comprendre au moins un dispositif de détection tel qu'une chambre d'ionisation et/ou un élément de diagnostic, permettant d'effectuer des mesures en vue de vérifier la conformation de la dose d'irradiation au volume cible.

Un tel dispositif présente l'avantage que la conformation au volume cible se fait sans l'utilisation de collimateurs variables et uniquement par un contrôle optimal du chemin de déplacement dudit spot. Le volume cible est découpé en plusieurs plans successifs perpendiculaires à la direction du faisceau, correspondant à des profondeurs successives, le déplacement du spot selon la profondeur d'un plan à l'autre se faisant en modifiant l'énergie du faisceau de particules.

Un autre objet de la présente invention réside dans un procédé de traitement d'un volume cible par un faisceau de particules, notamment de protons, issu d'un accélérateur à énergie fixe tel qu'un cyclotron, dans lequel on produit un spot étroit dirigé vers le volume cible à l'aide de ce faisceau de particules et en ce que l'on modifie l'énergie dudit faisceau de particules immédiatement après l'extraction de l'accélérateur. Ceci permet de traiter dans un environnement proche du cyclotron les problèmes de diffusion du faisceau, corrigés par exemple à l'aide de fentes, ou les problèmes de straggling corrigés directement à la sortie de l'accélérateur par un aimant d'analyse. Ceci permet également de diminuer le nombre de neutrons produits dans l'environnement proche du patient.

### Brève description de la figure

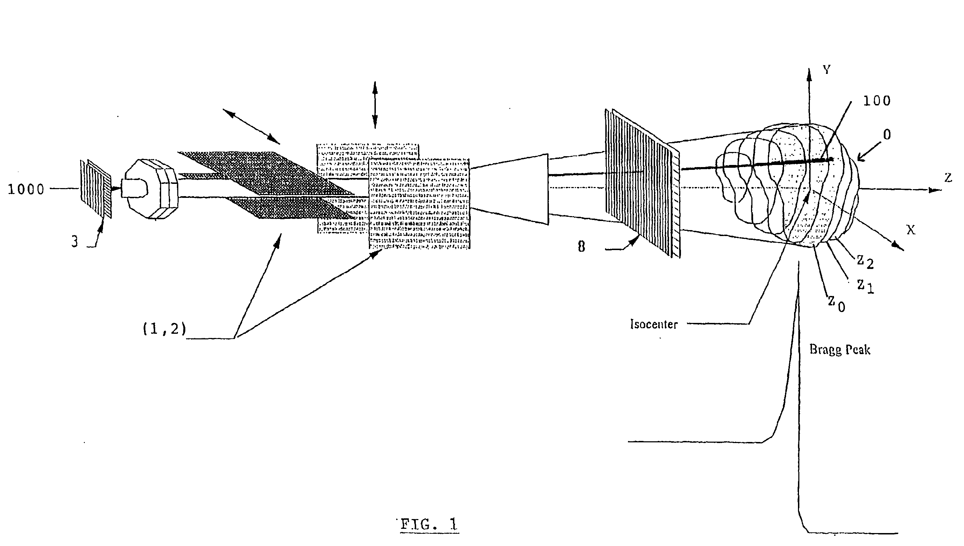

- La figure 1: représente une vue éclatée schématique du dispositif destiné à permettre l'irradiation pour le traitement d'un volume cible.

### Description d'une forme d'exécution préférée de l'invention

La présente invention vise à proposer un *dispositif* de traitement d'un faisceau de protons produit par un accélérateur, de préférence à énergie fixe, visant à permettre l'irradiation d'un volume cible constitué par exemple par une tumeur à traiter dans le cas d'un cancer, et qui présente des améliorations par rapport à l'état de la technique décrit à la figure 1.

Dans ce but, on vise à déplacer un spot produit à l'aide de ce faisceau de protons selon les trois dimensions directement dans le corps du patient afin de parcourir dans les trois dimensions le volume cible.

A la figure 1, on a représenté en partie le dispositif selon la présente invention. Selon une forme d'exécution préférée, un cyclotron (non représenté) est utilisé pour produire un faisceau de protons 1000 générant un spot 100 à déplacer.

On prévoit des moyens de variation permettant de modifier l'énergie du faisceau de protons immédiatement après son extraction de l'accélérateur pour permettre le déplacement du spot selon la dimension longitudinale, c'est-à-dire dans la direction du faisceau, afin de définir les différents plans successifs *Z*_{*0*}*, Z*_{*1*}*, Z*_{*2*} d'irradiation au sein du volume cible 0.

En effet, le volume cible 0 est découpé en plusieurs tranches successives correspondant à des profondeurs différentes. Chaque tranche ou chaque plan d'irradiation *Z*_{*0*}*, Z*_{*1*}*, Z*_{*2*} est ensuite balayé à l'aide des aimants de *scanning* 1 et 2 de nombreuses fois par ledit spot, ligne par ligne, de manière à couvrir une surface dont les contours seront généralement différents pour chaque tranche.

Les contours des surfaces à irradier sur chaque plan sont contrôlés par les aimants de balayage 1 et 2. Chacun de ces aimants permet d'effectuer un balayage soit dans la direction X, soit dans la direction Y.

En vue de modifier l'énergie du faisceau émis, on utilise de préférence un dégradeur d'énergie, et plus particulièrement un dégradeur d'énergie présentant des caractéristiques similaires à celles décrites dans la demande de brevet *WO00*/*38486* déposée par le Titulaire à ce sujet.

On observe ainsi de manière particulièrement avantageuse que le procédé et le dispositif selon la présente invention n'utilisent pas des éléments tels que des collimateurs, des compensateurs, des diffuseurs ou des modulateurs de parcours, ce qui allège particulièrement la mise en oeuvre dudit procédé.

En outre, on observe que selon la présente invention, aucun mouvement du patient n'est prévu. La procédure d'irradiation qui en résulte en sera allégée, plus rapide et plus précise. De ce fait, elle sera également moins coûteuse. On obtiendra ainsi une meilleure conformation de la dose délivrée au volume à traiter, et ceci en un temps minimum.

Selon une caractéristique particulièrement avantageuse, on observe que le déplacement du spot sur chaque plan d'irradiation se fait sans interruption du faisceau, ce qui permet un gain de temps considérable et diminue le risque de sous-dosage entre deux points d'irradiation consécutifs.

Selon la méthodologie mise en oeuvre, on prévoit de parcourir chaque plan à plusieurs reprises afin de limiter la dose délivrée point par point lors de chaque passage, ce qui augmente la sécurité tout en limitant les problèmes dus aux mouvements des organes à l'intérieur du corps comme la respiration.

De manière préférée, la dose délivrée lors de chaque passage représente environ 2% de la dose totale à délivrer.

En prévoyant de faire varier simultanément la vitesse de balayage du spot et l'intensité du faisceau de protons, on permet d'obtenir un ajustement de la dose à délivrer pour chaque élément de volume avec une flexibilité accrue.

En outre, on augmente également la sécurité de cette manière. En effet, tout problème lié à une imprécision de l'un des deux paramètres sera automatiquement corrigé par l'autre.

La méthodologie mise en oeuvre consiste à déterminer la dose correspondant à chaque spot en prédéterminant l'intensité du faisceau et la vitesse de balayage pour chaque volume d'irradiation (ou voxel), ceci à l'aide d'un système informatique de planification et de traitement. Au cours de l'irradiation, des cartes de doses sont établies en permanence à l'aide de mesures effectuées par des dispositifs de détection tels que des chambres d'ionisation 3,8 et autres éléments de diagnostic. L'intensité du faisceau et la vitesse de balayage seront instantanément recalculées et réajustées de manière à assurer que la dose prescrite soit effectivement délivrée dans le volume cible.

## Revendications

1. Dispositif de traitement d'un faisceau de particules produit par un accélérateur et vivant à permettre l'irradiation d'un volume cible (O) par un faisceau de particules (1000), ledit dispositif comprenant un accélérateur de particules tel qu'un cyclotron permettant de générer un spot (100) localisé dans la volume cible (O) associé à des moyens de balayage (1 et 2) permettant d'obtenir un balayage dudit spot (100) dans les deux directions (X,Y) perpendiculaires à la direction du faisceau (Z) et des moyens de variation permettant d'obtenir une variation de l'intensité dudit faisceau de particules, **caractérisé en ce qu'**il comprend des moyens de commande adaptés à commander de manière simultanée les moyens de balayage (1,2) et les moyens de variation de l'intensité du faisceau par un algorithme de planification et de régulation de manière à obtenir une conformation de la dose délivrée pour le volume cible (O).

2. Disposi tif selon la revendication 1, **caractérisé en ce que** les moyens de balayage (1,2) permettent le déplacement du spot (100) dans les deux directions (X, Y) perpendiculaires à la direction (Z) du faisceau de manière à définir un plan d'irradiation (Z0, Z1, Z2,...).

3. Dispositif selon la revendication 1 ou 2, **caractérisé en ce qu'**il comprend des moyens de variation de l'énergie du faisceau de particules (1000) qui permettent le déplacement du spot (100) au sein du volume (0) en passant d'un plan d'irradiation (Z0) à un autre (Z1, Z2,...) selon la profondeur.

4. Dispositif selon la revendication 2 ou 3, **caractérisé en ce que** les moyens de balayage (1, 2) permettent un balayage de chaque plan d'irradiation (Z0, Z1, Z2, ...) plusieurs fois par le spot (100).

5. Dispositif selon la revendication 3, **caractérisé en ce que** les moyens de variation de l'énergie du faisceau de particules (1000) sont disposés immédiatement après l'extraction de l'accélérateur du faisceau de particules (1000).

6. Dispositif Selon la revendication 5, **caractérisé en ce que** les moyens de variation de l'énergie du faisceau de particules (1000) sont constitués par un dégradeur d'énergie.

7. Dispositif selon l'une quelconque des revendications précédentes, **caractérisé en ce que** les moyens de balayage (1,2) sont constitués par deux aimants de scanning situés de préférence dans la tête d'irradiation.

8. Dispositif selon l'une quelconque des revendications précédentes, **caractérisé en ce que** les moyens de commande sont adaptés à exécuter la commande des moyens de balayage et des moyens de variation de l'intensité du faisceau à l'aide d'un algorithme de planification et de régulation des trajectoires desdites particules en y associant une boucle de régulation corrigeant en temps réel les trajectoires des particules du faisceau.

9. Dispositif selon l'une quelconque des revendications précédentes, **caractérisé en ce qu'**il comprend un système informatique pour la mise en oeuvre de l'algorithme de planification et de traitement qui permet de déterminer la dose correspondant à chaque spot en prédéterminant l'intensité du faisceau et la vitesse de balayage pour chaque volume d'irradiation ou voxel.

10. Dispositif selon l'une quelconque des revendications précédentes, **caractérisé en ce que** les moyens de balayage (1, 2) agissent en déplaçant le spot (100) sans interruption du faisceau (1000).

11. Dispositif selon l'une quelconque des revendications précédentes, **caractérisé en ce qu'**il comprend au moins un dispositif de détection tel qu'une chambre d'ionisation (3,8) et/ou un élément de diagnostic, permettant d'effectuer des mesures en vue de vérifier la conformation de la dose d'irradiation au volume cible (0).

## Claims

1. A device for treating a particle beam produced by an accelerator and aiming at allowing an irradiation of a target volume (0) by a particle beam (1000), said device comprising a particle accelerator such as a cyclotron allowing the generation of a spot (100) located within the target volume (0) associated with scanning means (1 and 2) for obtaining a scanning of said spot (100) in both directions (X,Y) perpendicular to the beam direction (Z) and variation means for obtaining an intensity variation of said particle beam, **characterized in that** it comprises control means adapted to control simultaneously the scanning means (1, 2) and the beam intensity variation means by a planning and regulation algorithm in order to obtain a conformation of the dose delivered to the target volume (0).

2. A device according to claim 1, **characterized in that** the scanning means (1, 2) allow the spot (100) to be moved in both directions (X,Y) perpendicular to the beam direction (Z) so as to define an irradiation plan (Z0, Z1, Z2, ...) .

3. A device according to claim 1 or 2,
**characterized in that** it comprises energy variation means for the particle beam (1000) allowing the spot (100) to be moved within the volume (0) by passing from an irradiation plan (Z0) to another one (Z1 Z2, ...) according to the depth.

4. A device according to claim 2 or 3,
**characterized in that** the scanning means (1, 2) allow a scanning of each irradiation plan (Z0, Z1, Z2, ...) several times by the spot (100).

5. A device according to claim 3, **characterized in that** the energy variation means for the particle beam (1000) are located immediately after the extraction of the particle beam (1000) from the accelerator.

6. A device according to claim 5, **characterized in that** the energy variation means for the particle beam (1000) consist of an energy degrader.

7. A device according to any of the preceding claims, **characterized in that** the scanning means (1, 2) consist of two scanning magnets preferably located in the irradiation head.

8. A device according to any of the preceding claims, **characterized in that** the control means are adapted to carry out the control of the scanning means and of the beam intensity variation means with the help of a planning and regulation algorithm for the trajectories of said particles by associating to it a regulation loop correcting in real time the trajectories of the beam particles.

9. A device according to any of the preceding claims, **characterized in that** it comprises a computer system for the implementation of the planning and treatment algorithm allowing the determination of the dose corresponding to each spot by predetermining the beam intensity and the scan rate for each irradiation volume or voxel.

10. A device according to any of the preceding claims, **characterized in that** the scanning means (1, 2) act by moving the spot (100) with no interruption of the beam (1000).

11. A device according to any of the preceding claims, **characterized in that** it comprises at least one detection device such as an ionization chamber (3, 8) and/or a diagnostic element, allowing measurements to be performed so as to check the conformation of the irradiation dose to the target volume (0).

## Patentansprüche

1. Vorrichtung zur Behandlung eines Teilchenstrahls, der von einem Beschleuniger erzeugt wird und die darauf abzielt, das Bestrahlen eines Zielvolumens (0) mit einem Teilchenstrahl (1000) zu erlauben, wobei die Vorrichtung einen Teilchenbeschleuniger, wie zum Beispiel ein Zyklotron umfasst, der es erlaubt, einen Spot (100) zu erzeugen, der sich in dem Zielvolumen (0) befindet, der mit Abtastmitteln (1 und 2) verbunden ist, die es erlauben, ein Abtasten des Spots (100) in die zwei Richtungen (X, Y) senkrecht zu der Richtung des Strahls (Z) zu erhalten, und Mittel zum Variieren, die es erlauben, eine Variation der Intensität des Teilchenstrahls zu erzielen, **dadurch gekennzeichnet, dass** sie Steuermittel aufweist, die gleichzeitig die Abtastmittel (1, 2) und die Mittel zum Variieren der Strahlintensität durch einen Planungs- und Regelalgorithmus steuern können, um eine Anpassung der abgegebenen Dosis an das Zielvolumen (0) zu erzielen.

2. Vorrichtung nach Anspruch 1, **dadurch gekennzeichnet, dass** die Abtastmittel (1, 2) das Bewegen des Spots (100) in die zwei Richtungen (X, Y) senkrecht zu der Richtung (Z) des Strahls erlauben, um eine Bestrahlungsebene (Z0, Z1, Z2, ...) zu bestimmen.

3. Vorrichtung nach Anspruch 1 oder 2, **dadurch gekennzeichnet, dass** sie Mittel zum Variieren der Energie des Teilchenstrahls (1000) umfasst, die das Bewegen des Spots (100) innerhalb des Volumens (0) erlauben, indem sie von einer Bestrahlungsebene (Z0) auf eine andere (Z1, Z2, ...) gemäß der Tiefe übergehen.

4. Vorrichtung nach Anspruch 2 oder 3, **dadurch gekennzeichnet, dass** die Abtastmittel (1, 2) ein Abtasten jeder Bestrahlungsebene (Z0, Z1, Z2, ...) mehrmals durch

5. Vorrichtung nach Anspruch 3, **dadurch gekennzeichnet, dass** die Mittel zum Variieren der Energie des Teilchenstrahls (1000) unmittelbar nach dem Extrahieren aus dem Beschleuniger des Teilchenstrahls (1000) angeordnet sind.

6. Vorrichtung nach Anspruch 5, **dadurch gekennzeichnet, dass** die Mittel zum Variieren der Energie des Teilchenstrahls (1000) aus einem Energieabstufer bestehen.

7. Vorrichtung nach irgendeinem der vorhergehenden Ansprüche, **dadurch gekennzeichnet, dass** die Abtastmittel (1, 2) aus zwei Scanningmagneten bestehen, die vorzugsweise in dem Bestrahlungskopf liegen.

8. Vorrichtung nach irgendeinem der vorhergehenden Ansprüche, **dadurch gekennzeichnet, dass** die Steuermittel das Steuern der Abtastmittel und der Mittel zum Variieren der Intensität des Teilchenstrahls mit Hilfe eines Planungs- und Regelalgorithmus der Bahnen der Teilchen ausführen können, indem damit ein Regelkreis verbunden wird, der in Echtzeit die Bahnen der Teilchen des Strahls korrigiert.

9. Vorrichtung nach irgendeinem der vorhergehenden Ansprüche, **dadurch gekennzeichnet, dass** sie ein EDV-System für das Umsetzen des Planungs- und Verarbeitungsalgorithmus aufweist, das es erlaubt, die Dosis zu bestimmen, die jedem Spot entspricht, indem im Voraus die Strahlintensität und die Abtastgeschwindigkeit für jedes Bestrahlungsvolumen oder Voxel bestimmt werden.

10. Vorrichtung nach irgendeinem der vorhergehenden Ansprüche, **dadurch gekennzeichnet, dass** die Abtastmittel (1, 2) wirken, indem sie den Spot (100) ohne Unterbrechung des Strahls (1000) bewegen.

11. Vorrichtung nach irgendeinem der vorhergehenden Ansprüche, **dadurch gekennzeichnet, dass** sie mindestens ein Nachweisgerät aufweist, wie zum Beispiel eine Ionisationskammer (3,8) und/oder ein Diagnoseelement, das es erlaubt, Messungen zum Prüfen der Anpassung der Bestrahlungsdosis an das Zielvolumen (0) durchzuführen.
